# EUROPEAN PATENT SPECIFICATION

(11) **EP 0 665 843 B1**
(45) Date of publication and mention of the grant of the patent: **13.08.2003**
(21) Application number: 93922406.9
(22) Date of filing: 30.09.1993
(51) Int. Cl.: C07D 453/02, A61K 31/435

(54) **SUBSTITUTED QUINUCLIDINES AS SUBSTANCE P ANTAGONISTS**
SUBSTITUIERTE CHINUCLIDINE ALS SUBSTANZ P ANTAGONISTEN
QUINUCLIDINES SUBSTITUEES UTILES COMME ANTAGONISTES DE LA SUBSTANCE P

(30) Priority: 28.10.1992 JP 29056992
(43) Date of publication of application: 09.08.1995
(73) Proprietor: PFIZER INC., New York, N.Y. 10017 (US)
(72) Inventor: ITO, Fumitaka, Chiga-gun, Aichi 470-23 (JP); KOKURA, Toshihide, Handa, Aichi 475 (JP); NAKANE, Masami, Nagowa, Aichi 466 (JP); SATAKE, Kunio, Handa, Aichi 475 (JP); WAKABAYASHI, Hiroaki, Kariya, Aichi 448 (JP)
(74) Representative: Eddowes, Simon
(86) International application number: US9309169
(87) International publication number: WO94010170

(56) References cited:
- WO-A-90/05729
- WO-A-92/20676

## Description

### Technical Field

This invention relates to novel and useful substituted quinuclidine compounds of interest to those in the field of medical chemistry and chemotherapy. More particularly, it is concerned with a novel series of substituted 3-aminoquinuclidines, including their pharmaceutically acceptable salts, which are of special value in view of their ability to antagonize substance P. In this way, these compounds are of use in treating gastrointestinal disorders, central nervous system disorders, inflammatory diseases, asthma, pain and migraine.

### Background Art

E. J. Warawa in United States Patent No. 3560510 discloses certain 3-amino-2-benzhydrylquinuclidines as being useful as diuretic agents, with the corresponding unsubstituted 3-benzylamino compounds acting as intermediates for same. Additionally, E. J. Warawa et al. in the Journal of Medicinal Chemistry, Vol.18, p.587 (1975) extends this work to other members of the series wherein the 3-amino moiety is either ethylamino, β-phenylethylamino, β-isopropylamino, or 2-furfurylamino, but in no instance is there any substitution on the phenyl group itself.

Furthermore, neither of the aforementioned documents teaches or suggests any of these compounds to be useful as substance P antagonists.

Substance P is a naturally occurring undecapeptide belonging to the tachykinin family of peptides, the latter being so-named because of their prompt stimulatory action on smooth muscle tissue. More specially, substance P is a pharmaceutically active neuropeptide that is produced in mammals (having originally been isolated from gut) and possesses a characteristic amino acid sequence that is illustrated by D. F. Veber et al. in United States Patent No. 4680283. The wide involvement of substance P and other tachykinins in the pathophysiology of numerous diseases has been amply demonstrated in the art. For instance, substance P has recently been shown to be involved in the transmission of pain or migraine (see B. E. B. Sandberg et al., Journal of Medicinal Chemistry, Vol. 25, p.1009 (1982)), as well as in central nervous system disorders such as anxiety and schizophrenia, in respiratory and inflammatory diseases such as asthma and rheumatoid arthritis, respectively, and in gastrointestinal disorders and diseases of GI tract, like ulcerative colitis and Crohn's diseases, etc (see D. Regoli in "Trends in Cluster Headache" edited by F. Sicuteri et al., Elsevier Scientific Publishers, Amsterdam, 1987, PP. 85-95).

In the recent past, some attempts have been made to provide peptide-like substances that are antagonists for substance P and other tachykinin peptides in order to more effectively treat the various disorders and diseases listed above. The peptide-like nature of such substances makes them too labile from a metabolic point of view to serve as practical therapeutic agents in the treatment of disease. The non-peptidic antagonists of the present invention, on the other hand, do not possess this drawback, being far more stable from a metabolic point of view than the previously-discussed prior art agents.

Compounds of similar structure and similar pharmacological activity to the object compounds of the present invention are described in WO 90/05729 and WO 92/20676.

WO 90/05729 discloses a series of cis-3-[(cyclic)methylamino]-2-[(α-substituted)-arylmethyl]quinuclidines including 2-benzhydryl derivatives, 2-substituted benzhydryl derivatives (wherein the substituents were alkyl, alkoxy, halogen and the like), 2-(bis-(2-thienyl)methyl) derivatives and the like.

WO 92/20676 discloses mainly a series of 3-[2-methoxy-5-(substituted)benzyl-amino]-2-benzhydryl quinuclidines including 4-alkenyl derivatives, 6-phenethyl derivatives, 5- and 6-dialkylaminocarbonyl derivatives, 5-dialkylaminoalkyl derivatives, 6-hydroxyalkyl derivatives, 5-alkylaminocarbonyl derivatives, 5-aminocarbonyl derivatives, 5-carboxyl derivatives, 5- and 6-alkoxycarbonyl derivatives, 5-(N-alkoxy-N-alkyl)aminocarbonyl derivatives, 5-morpholinocarbonyl derivatives and the like. Additionally, the quinuclidine compounds disclosed in WO 92/20676 have various kind of substituents also at 5-position on the benzylamino moiety i.e alkoxy (methoxy), alkyl (isopropyl), alkylthio (methylthio), halosubstituted alkoxy (trifluoromethoxy), halogen, alkylsulfinyl (methylsulfinyl), dialkylamino (dimethylamino) and the like. Further-more, it shows that compounds disclosed in both WO 90/05729 and WO 92/20676 have activity as substance P antagonists, anti-inflammatory activity and anti-psychotic activity.

The present inventors have worked to prepare compounds useful as substance P antagonist, and after extensive research, have succeeded in synthesizing a series of compounds as will be disclosed in detail herein.

### Brief Disclosure of the Invention

The present invention provides novel substituted quinuclidine derivatives of the following chemical formula I and its pharmaceutically acceptable salt: 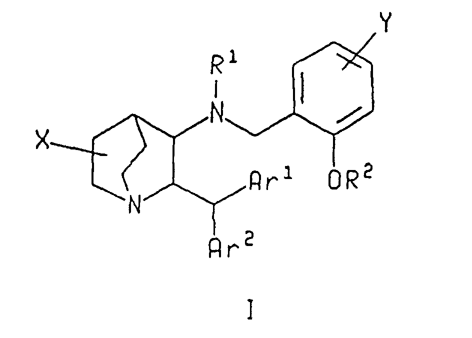 wherein Ar¹ and Ar² are each, independently, thienyl, phenyl, fluorophenyl, chlorophenyl or bromophenyl; X is -CONR³R⁴, -CO₂R³, -CH₂OR³, -CH₂NR³R⁴. or -CONR³OR⁴; R¹, R³ and R⁴ are each, independently, hydrogen or alkyl having 1 to 4 carbon atoms; R² is alkyl having 1 to 4 carbon atoms; Y is alkenyl having 2 to 4 carbon atoms.
with the proviso that when R¹ is hydrogen, and Ar¹ and Ar² are both phenyl, R² is not an alkyl group having 1 carbon atom.

The compounds of formula I show pharmaceutical activity as substance P antagonists. Therefore they are useful for treatment or prevention of a condition selected from the group consisting of inflammatory diseases (e.g., arthritis, psoriasis, asthma and inflammatory bowel disease), anxiety, depression or dysthymic disorders, colitis, psychosis, pain, gastroesophageal reflux disease, allergies such as eczema and rhinitis, chronic obstructive airways disease, hypersensitivity disorders such as poison ivy, vasospastic disease such as angina, migraine and Reynaud's disease, fibrosing and collagen diseases such as scleroderma and eosinophilic fascioliasis, reflex sympathetic dystrophy such as shoulder/hand syndrome, addiction disorders such as alcoholism, stress related somatic disorders, peripheral neuropathy, neuralgia, neuropathological disorders such as Alzheimer's disease, AIDS related dementia, diabetic neuropathy and multiple sclerosis, disorders related to immune enhancement or suppression such as systemic lupus erythematosus, and rheumatic diseases such as fibrositis in a mammal, including a human.

Accordingly, the present invention includes pharmaceutical compositions for antagonizing mammal's substance P which comprises a pharmaceutically acceptable carrier or diluent and a compound of formula I or a pharmaceutically acceptable salt thereof. These pharmaceutical compositions are useful for treating or preventing one of the aforementioned conditions, in a mammals, including a human.

Compounds of the present invention may be used for the preparation of medicaments for antagonizing substance P in a mammalian subject.

### Detailed Disclosure of the Invention

In this specification:
the term "alkyl" is used herein to mean straight or branched hydrocarbon chain radicals including, but not limited to, methyl, ethyl, n-propyl, isopropyl, n-butyl, isobutyl, t-butyl, and the like;
the term "alkenyl" is used herein to mean straight or branched hydrocarbon chain radicals having one double bond including, but not limited to, ethenyl, 1- and 2-propenyl, 2-methyl-1-propenyl, 1- and 2-butenyl and the like;
the term "alkynyl" is used herein to mean straight or branched hydrocarbon chain radicals having one triple bond including, but not limited to, ethynyl, propynyl, butynyl and the like;
the term "halosubstituted alkyl" refers to an alkyl radical substituted with one or more halogens including, but not limited to, chloromethyl, bromoethyl, trifluoromethyl and the like;
the term "alkylsulfonyl" is used herein to mean -SO₂R⁵ (R⁵ is alkyl) including, but not limited to, methylsulfonyl, ethylsulfonyl, n-propylsulfonyl, isopropylsulfonyl, n-butyl sulfonyl, isobutylsulfonyl, t-butylsulfonyl and the like;
the term "alkylamino" is used herein to mean -NHR⁶ (R⁶ is alkyl) including, but not limited to, methylamino, ethylamino, n-propylamino, isopropylamino, n-butyl-amino, t-butylamino and the like;
the term "alkanoylamino" is used herein to mean -NHCOR⁷ (R⁷ is alkyl or halosubstituted alkyl) including, but not limited to, formylamino, acetylamino, propionylamino, butyrylamino, isobutyrylamino, trifluoroacetylamino and the like;
the term "alkylsulfonylamino" is used herein to mean -NHSO₂R⁸ (R⁸ is alkyl or halosubstituted alkyl) including, but not limited to, methylsulfonylamino, ethylsulfonylamino, trifluoromethylsulfonylamino and the like;
the term "N-alkyl-N-alkylsulfonylamino" is used herein to mean -N(R⁹)SO₂R¹⁰ (R⁹ is alkyl and R¹⁰ is alkyl or halosubstituted alkyl) including, but not limited to, N-methyl-N-methylsulfonylamino, N-ethyl-N-methylsulfonylamino, N-n-propyl-N-methylsulfonylamino, N-isopropyl-N-methyl sulfonylamino, N-methyl-N-trifluoromethylsulfonylamino, N-ethyl-N-trifluoromethylsulfonylamino, N-n-propyl-N-trifluoromethylsulfonylamino, N-isopropyl-N-trifluoromethyl sulfonylamino; and
the term "N-alkyl-N-alkanoylamino" is used herein to mean -N(R¹¹)COR¹² (R¹¹ is alkyl and R¹² is alkyl or halosubstituted alkyl) including, but not limited to, N-acetyl-N-methylamino, N-acetyl-N-ethylamino, N-acetyl-N-n-propylamino, N-acetyl-N-isopropylamino,N-trifluoroacetyl-N-methylamino,N-trifluoroacetyl-N-ethylamino,N-trifluoro-acetyl-N-n-propylamino, N-trifluoroacetyl-N-isopropylamino.

The preferred group for Ar¹ and Ar² is phenyl - i.e., the preferred group for Ar¹-CH-Ar² is diphenylmethyl.

A particularly preferred sub-group of compounds of this invention consists of the compounds of formula I, wherein Ar¹ and Ar² are each phenyl, R¹ is hydrogen, R² is methyl, X is at the 3-position of the quinuclidine ring and X is either carboxy or aminocarbonyl. Within this particularly preferred sub-group, especially preferred compounds are compounds in which Y is isopropenyl.

One preferred individual compound of this invention is (3R,4S,5S,6S)-5-(5-Isopropenyl-2-methoxybenzylamino)-6-diphenylmethyl-1-azabicyclo[2.2.2]octane-3-carboxamide and 3-carboxylic acid derivative.

The novel compounds of the present invention can be prepared by the following methods. The compounds of formula I may be prepared by a number of synthetic methods well known by those skilled in the art. See, for instance, WO 92/20676. Thus, typically, the methods in the following scheme can be employed to prepare the objective compounds of the present invention. 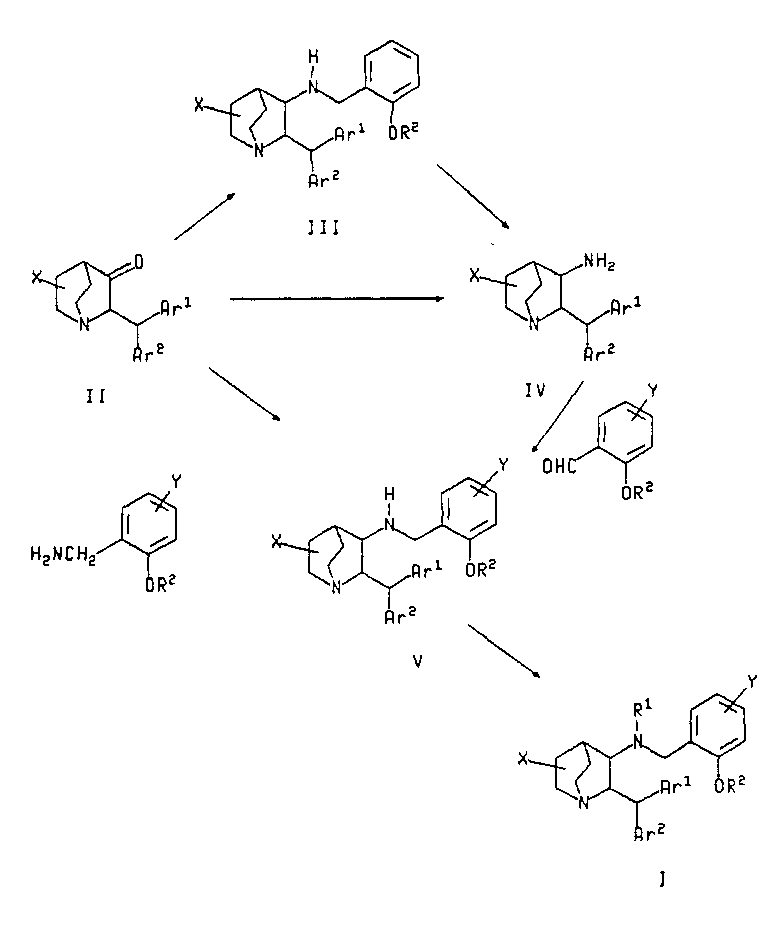 (wherein all the symbols are the same as in formula I).

### 1st STEP [ compound II to IV ]:

The first step is through the introduction of a substituted benzylamine (e.g. 2-alkoxy benzylamine etc.) into 3-position of 2-diarylmethyl quinuclidin-3-one II and the subsequent release of the benzyl group from 3-benzylaminoquinuclidine III by using hydrogenation etc. to afford a requisite intermediate, 2-diarylmethylquinuclidin-3-amine IV.

The 2-diarylmethylquinuclidin-3-one II can be synthesized according to the similar manner with methods including prior aryliden-formation and subsequent 1,4-addition reaction described in WO 92/20676. The compound II can be converted to the compound III according to the procedure including reductive amination with a substituted benzylamine well described in WO 92/20676.

The subsequent conversion of the compound III to the compound IV involves reduction by catalytic hydrogenation (i.e. Pearlman's catalyst etc.) or using hydride reagents such as aluminum-based reagents, boranes, borohydrides or trialkylsilanes. In most of the cases, catalytic hydrogenation can be carried out in a suitable organic solvent under hydrogen atmosphere at room temperature for a few hours to a few days, in the presence of a catalyst.

### 2nd STEP [ compound IV to V ]:

The second step is through the condensation of 3-amino-2-diarylmethylquinuclidine IV and a substituted benzaldehyde followed by reduction. In this second step, the direct arylmethylation can be carried out by the reductive amination [e.g. sodium cyanoborohydride in methanol; Journal of American Chemical Society, 93, 2897 (1971)] to the objective compound V. Several other reducing reagents such as NaBH₄, NaBH(OAc)₃ or trialkylsilanes can be also used to perform this transformation.

Alternatively, the reductive amination of compound II with a corresponding substituted benzylamine can be also employed in order to obtain the objective compound V (see WO 92/20676).

In case of preparing the substituted benzaldehyde, the standard methods (formylation of a substituted alkoxybenzene) well known by those skilled in the art in the following literature can be used: (A) Duffs reaction (hexamethylenetetramine /TFA), Synthetic Communication, 15, 61 (1985), (B) TiCl₄ / dichloromethylether, Journal of Organic Chemistry, 51, 4073 (1986), (C) A process by two step reaction (HCI, HCHO, then 2-nitropropane, NaOMe), JP-58-501127 and (D) Journal of the American Chemical Society, 2466, (1955).

Additionally, in order to prepare the alkoxybenzaldehyde wherein substituents are alkenyl or alkynyl, a Pd-catalyzed coupling reaction with halosubstituted alkoxybenzene as described in the following literature can be employed: (E) Angewandte Chemie International Edition in English, 25, 508 (1986) by J. K. Stille et al., (F) Journal of Organic Chemistry, 53, 1170 (1988) by J. K. Stille et al., (G) Tetrahedron Letters, 4467 (1975) by K. Sonogashira et al., (H) Synthesis, 627 (1980) by N. Hagihara et al..

For example, corresponding alkenyltin compounds or alkynyltin compounds are reacted with 5-halogen-o-anisaldehyde in a reaction inert solvent in the presence of a suitable palladium catalyst. In this reaction, preferred palladium catalysts are Pd(PPh₃)₄ or PdCl₂(PPh₃)₂. Reaction temperature is usually in the temperature range of room temperature through reflux temperature and the reaction is usually completed within 1 hr to 48 hr. In order to obtain 5-alkenyl-2-alkoxybenzaldehyde, preferred solvents are DMF, THF, toluene and the like. In order to obtain 5-alkynyl-2-alkoxy-benzaldehyde, preferred solvents are diethylamine, piperidine, triethylamine and the like.

In addition, in case of alkylsulfonyl for Y in the above formula, 5-halogen-o-anisaldehyde can be used as a starting compound. First, the 5-position of the starting compound is converted into 5-alkylthio, and then into 5-alkylsulfonyl to afford the objective intermediate, 5-alkylsulfonyl-o-anisaldehyde. Cyclic acetalization is required in the reaction route in order to protect aldehyde group in the above anisaldehyde. The said 5-alkylthio intermediate can be also synthesized by a method described in Bulletin of the Chemical Society of Japan, 51, 2435 (1978) by M. Ando et al..

Furthermore, in case of halosubstituted alkyl for Y in the above formula, 4-(halosubstituted alkyl)phenol can be used as a starting compound. The starting compound is alkylated with alkyl halide in the presence of a suitable base. Subsequently, the resulting alkoxybenzene is formylated by a standard method to afford the objective intermediate, 5-halosubstituted alkyl-o-anisaldehyde.

Additionally, in case of alkylamino for Y in the above formula, 3-(1,3-dioxolan-2-yl)-4-methoxyaniline can be used as a starting compound. The starting compound is reacted with a corresponding aldehyde in the presence of a suitable reducing agent (i.e. NaCNBH₄ etc.) by a method of reductive alkylation to give the objective intermediate, 5-alkylamino-o-anisaldehyde. This procedure is well described in Journal of the American Chemical Society, 2897 (1971) by R. F. Borch et al..

In case of N-alkyl-N-alkylsulfonylamino, N-alkyl-N-alkanoylamino, alkanoyl-amino or alkylsulfonylamino for Y in the above formula, 5-nitrosalicylaldehyde can be used as a starting compound. First, the hydroxyl group in 5-nitro salicylaldehyde is alkylated with a suitable alkylating agent (e.g. methyl iodide etc.). Subsequently, after the aldehyde group in the above 5-nitroaldehyde is cyclic-acetalized with a suitable acid catalyst (i.e. camphor sulfonic acid (CSA), p-toluene sulfonic acid etc.), the nitro group in the 5-nitro derivative is converted into an amino group with a suitable catalyst such as PtO₂. The amino group in the 5-amino derivative is converted into the respective corresponding functional groups to afford the objective substituted aldehyde. The methods of alkylation, acylation and alkylsulfonyl formation employed in that conversion are the methods well known by those skilled in the art. These methods are described in detail in the following examples.

As mentioned above, synthetic methods for the alkoxy benzaldehyde intermediates wherein Y is substituted at 5-position on 2-alkoxybenzaldehyde and R² is methyl are mainly described. However, it is a matter of course that the similar methods can be applicable also to the intermediates wherein Y is substituted at other position on 2-alkoxy benzaldehyde or R² is alkyl groups other than methyl such as ethyl, n-propyl, isopropyl, n-butyl, isobutyl, t-butyl and the like.

As described in WO 92/20676, a substituent group in compound V can be converted to various different substituent groups. For example, a compound having carboxyl, methoxycarbonyl, hydroxymethyl, alkylaminomethyl or dialkylamino-methyl as X can be synthesized from the corresponding carboxamide derivative by methods well known to those skilled in the art.

Additionally, several other carboxamide derivatives, such as those in which X is morpholinocarbonyl, thiamorpholinocarbonyl, N-methyl-N-methoxyaminocarbonyl, N-ethyl-carboxamide, can be synthesized from the corresponding carboxylic acid or carboxamide by utilizing the process documented in WO 92/20676.

In addition, the substituted quinuclidine of the present invention wherein R¹ is alkyl can be synthesized by the reductive alkylation (well described in Journal of the American Chemical Society, 2897 (1971) by R. F. Borch et al.) of the substituted quinuclidine V, wherein R¹ is hydrogen, prepared by the above mentioned methods. Particularly, it can be synthesized by reacting compound V with an aldehyde having corresponding carbon atoms in acetic acid/methanol in the presence of a suitable reducing agent such as NaCNBH₃.

Additionally, (+)-(3R,4R)- and (-)-(3S,4S)-N,N-diethyl-5-oxo-1-azabicyclo[2.2.2]octane-3-carboxamide (see WO 92/20676) are useful for preparing single isomers of the compounds of formula I.

The compounds of formula I can be isolated and purified by conventional procedures, such as recrystallisation or chromatographic purification.

Inasmuch as the quinuclidine compounds of this invention all possess at least four asymmetric center, they are capable of occurring in various stereoisomeric forms or configurations. The present invention is meant to include all such forms within its scope. For instance, diastereomeric mixtures can be separated by methods well known to those skilled in the art, e.g., by fractional crystallization, column chromatography and the like. Individual diastereomers can also be synthesized by resolving racemic mixtures of formula I using standard procedures of organic chemistry.

Insofar as the quinuclidine compounds of this invention are basic compounds, they are all capable of forming a wide variety of different salts with various inorganic and organic acids. Although such salts must be pharmaceutically acceptable for administration to animals, it is often desirable in practice to initially isolate the quinuclidine base compound from the reaction mixture as a pharmaceutically unacceptable salt and then simply convert to the free base compound by treatment with an alkaline reagent and thereafter, subsequently convert the free base to a pharmaceutically acceptable acid addition salt. The acid addition salts of the quinuclidine base compounds of this invention are readily prepared by treating the base compound with a substantially equivalent amount of the chosen mineral or organic acid in an aqueous solvent or in a suitable organic solvent, such as methanol or ethanol. Upon careful evaporation of the solvent, the desired solid salt is readily obtained.

The acids which are used to prepare the pharmaceutically acceptable acid addition salts of the quinuclidine base compounds of this invention are those which form non-toxic acid addition salts, i.e., salts containing pharmaceutically acceptable anions, such as the hydrochloride, hydrobromide, hydroiodide, nitrate, sulfate or bisulfate, phosphate or acid phosphate, acetate, lactate, citrate or acid citrate, tartrate or bitartrate, succinate, maleate, fumarate, gluconate, saccharate, benzoate, methane sulfonate, ethanesulfonate, benzenesulfonate, p-toluene sulfonate and pamoate [i.e., 1.1'-methylene-bis-(2-hydroxy-3-naphthoate)] salts.

Some quinuclidine compounds of the invention which have also acidic groups are capable of forming base salts with various pharmaceutically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the herein described acidic quinuclidine derivatives. These particular non-toxic base salts include those derived form such pharmaceutically acceptable cations as sodium, potassium, calcium and magnesium, etc. These salts can easily be prepared by treating the aforementioned acidic quinuclidine compounds with an aqueous solution containing the desired pharmaceutically acceptable cation, and then evaporating the resulting solution to dryness, preferably under reduced pressure. Altematively, they may also be prepared by mixing lower alkanoic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum production of yields of the desired final product.

The quinuclidine compounds of the present invention exhibit significant substance P receptor-binding activity and therefore, are of value in the treatment of a wide variety of clinical conditions which are characterized by the presence of an excess of said substance P activity. Such conditions include gastrointestinal disorders such as ulcer and colitis and other like diseases of the gastrointestinal tract, central nervous system disorders such as anxiety and psychosis, inflammatory diseases such as rheumatoid arthritis and inflammatory bowel diseases, respiratory diseases such as asthma, as well as pain in any of the aforesaid conditions, including migraine. Hence, these compounds are readily adapted to therapeutic use as substance P antagonists for the control and/or treatment of any of the aforesaid clinical conditions in mammals, including humans.

Compounds of the present invention, when tested as an antiinflammatory agent, exhibit a significant degree of activity in the mustard oil-induced rat foot edema test [described by F. Lembeck et al., British Journal of Pharmacology, Vol.105, P.527 (1992)] and the capsaicin-induced plasma extravasation test in guinea pig ureter [described by A. Nagahisa et al., European Journal of Pharmacology, Vol.217, P.191 (1992)].

The radiolabelled quinuclidine compounds of the above formula are useful as research and diagnostic tools in metabolism pharmacokinetic studies and in binding assays with the drug in both animal and human. Specific applications in research include radioligand binding assays, autoradiography studies and in vivo binding studies, while specific applications in the diagnostic area include studies of substance P receptor in the human brain, such as up/down regulation in a diseases state, and in vivo binding in the relevant tissues for inflammation, e.g., immune-type cell or cells that are directly involved in inflammatory bowel disorders and the like. Specifically, the radiolabelled forms of the quinuclidine compounds are the tritium and ¹⁴C-isotopes of the substituted quinuclidine in this invention.

The quinuclidine compounds of formula I can be administered to a mammalian subject, e.g., a human subject, via either the oral, parenteral or topical routes. In general, these compounds are most desirably administered to a human subject in doses ranging from about 1 to 300mg per day, although variations will necessarily occur depending upon the weight and condition of the subject being treated and the particular route of administration chosen. However, a dosage level that is in the range of about 0.06mg to about 6mg per kg of body weight per day is most desirably employed. Variations will occur depending upon the potency of the compound administered and the individual response to said medicament, as well as the severity of the condition being treated and the type of pharmaceutical formulation chosen and the time period and interval at which such administration is carried out. In some instances, dosage levels below the lower limit of the aforesaid range may be more than adequate, while in other cases still larger doses may be employed without causing any harmful side effects provided that such higher dose levels are first divided into several small doses for administration throughout the day.

The compounds of the present invention may be administered alone or in combination with pharmaceutically acceptable carriers or diluents by either of the three routes previously indicated, and such administration can be carried out in single or multiple doses. More particularly, the novel therapeutic agents of the invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups and the like. Such carriers include solid diluents or fillers, sterile aqueous media and various non-toxic organic solvents, etc. Moreover, oral pharmaceutical compositions can be suitably sweetened and/or flavored. In general, the therapeutically-effective compounds of this invention are present in such dosage forms at concentration levels ranging about 5.0% to about 70% by weight.

For oral administration, tablets containing various excipients such as microcrystalline cellulose, sodium citrate, calcium carbonate, dicalcium phosphate and glycine may be employed along with various disintegrants such as starch and preferably corn, potato or tapioca starch, alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatine capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered (preferably pH )8) if necessary and the liquid diluent first rendered isotonic. These aqueous solutions are suitable for intravenous injection purposes. The oily solutions are suitable for intra-articular, intramuscular and subcutaneous injection purposes. The preparation of all these solutions under sterile conditions is readily accomplished by standard pharmaceutical techniques well-known to those skilled in the art. Additionally, it is also possible to administer the compounds of the present invention topically when treating inflammatory conditions of the skin and this may preferably be done by way of creams, jellies, gels, pastes, ointments and the like, in accordance with standard pharmaceutical practice.

The activity of the compounds of the present invention, as substance P antagonists, is determined by their ability to inhibit the binding of substance P at its receptor sites in bovine caudate tissue or IM-9 cells employing radioactive ligands. The substance P antagonist activity of the herein described quinuclidine compounds is evaluated by using the standard assay procedure described by M. A. Cascieri et al., as reported in Journal of Biological Chemistry, Vol.258, p.5158 (1983). This method essentially involves determining the concentration of the individual compound required to reduce by 50% the amount of radiolabelled substance P ligands at their receptor sites in said isolated cow tissues or IM-9 cells, thereby affording characteristic IC₅₀ values for each compound tested. In this test, some preferred compounds indicated low IC₅₀ values of in the range of 0.1 to 1.9 nM, with respect to inhibition of binding at its receptor.

The anti-inflammatory activity of the compounds of the present invention is demonstrated in the previously mentioned mustard oil-induced rat foot edema test. In this test, anti-inflammatory activity is determined as the percent inhibition of plasma protein extravasation in the hind paw of female Sprague-Dawley rats (weighing 100-150g) in response to the application of mustard oil to the dorsal skin. The compounds of the present invention are dissolved in 0.1% methyl cellulose/water and dosed orally 1h before mustard oil challenge. After Evans Blue injection solution (50mg/kg, dissolved in saline including 0.02% bovine serum albumin) is injected intravenously, rat's hind paw is painted with 5% mustard oil in paraffin oil and 20min later the foot is amputated, frozen, pulverized and the Evans Blue dye is extracted and determined colorimetrically.

Alternatively, the antiinflammatory activity of the compounds of the present invention is demonstrated by a capsaicin-induced plasma extravasation test. In this test, antiinflammatory activity is determined as the percent inhibition of plasma protein extravasation in the ureter of male Hartley guinea pigs (weighing 450-500g) in response to the intraperitoneal injection of capsaicin into anesthetized animals. The compounds of the present invention are dissolved in 0.1% methyl cellulose/water and dosed orally 1h before capsaicin challenge. Evans Blue dye (30mg/kg) is administered intravenously 5 min before capsaicin challenge. The animals are killed 10min after capsaicin injection and both right and left ureters are removed. The Evans Blue dye is extracted and determined colorimetrically.

In the mustard oil-induced rat foot edema test and capsaicin-induced plasma extravasation test, compounds are considered active if the difference in response between the drug-treated animals and a control group receiving the vehicle alone is statistically significant. In those test, some preferred compounds indicated high percentage with respect to inhibition of plasma protein extravasation.

The anti-psychotic activity of the compounds of the present invention as neuroleptic agents for the control of various psychotic disorders is determined primarily by a study of their ability to suppress substance P-induced hypermotility in rats. This study is carried out by first dosing the rats with a control compound or with an appropriate test compound of the present invention, then injecting the rats with substance P by intra-cerebral administration via canula and thereafter measuring their individual locomotor response to said stimuli.

### EXAMPLES

The present invention is illustrated by the following examples. However, it should be understood that the invention is not limited to the specific details of these examples. Proton nuclear magnetic resonance spectra (NMR) were measured in CDCl₃ at 270MHz unless otherwise indicated and peak positions are expressed in parts per million (ppm) downfield from tetramethylsilane. The peak shapes are denoted as follows: s, singlet; d, doublet; t, triplet; m, multiplet; br, broad.

### Comparative Example 1

### (3R,4S,5S,6S)-6-Diphenylmethyl-5-(2-methoxy-5-methylsulfonylbenzylamino)-1 -aza-bicyclo[2.2.2]octane-3-carboxamide dihydrochloride

### Synthesis of 5-methylsulfonyl-o-anisaldehyde

A solution of 5-bromo-o-anisaldehyde (49.7 g, 231 mmol), ethylene glycol (17.2 g, 277 mmol) and p-toluene sulfonic acid (4.3 g, 23 mmol) in dry toluene (400 ml) was heated at reflux for 21 hr. And then ethylene glycol (4.3 g, 70 mmol) was added to the reaction mixture and heated at reflux for 6 hr. The resulting solution was washed with sat. NaHCO₃, water and brine, dried over MgSO₄ and concentrated in vacuo to give crude 2-(5-bromo-2-methoxyphenyl)-1,3-dioxolane (75.7 g) as a dark red oil. The residue was added NaHCO₃ and then distilled to give 2-(5-bromo-2-methoxyphenyl)-1,3-dioxolane (52.8 g, 88.3%) as a colorless oil.
¹H NMR (CDCl₃) δ: 7.64 (d, J=2.6Hz, 1H), 7.42 (dd, J=2.6, 8.8Hz, 1H), 6.78 (d, J=8.8Hz, 1H), 6.10 (s, 1H), 4.16-4.00 (m, 4H), 3.85 (s, 3H).

To a stirred cooled (-78°C) solution of 2-(5-bromo-2-methoxyphenyl)-1,3-dioxolane (51.13 g, 197.3 mmol) in THF (300 ml) was added dropwise a 1.6M-n-butyl lithium (185 ml, 296 mmol) under N₂ atmosphere. And the mixture was stirred at -78°C for 2.5 hr. To the reaction mixture was added dropwise dimethyl disulfide (23.1 ml, 256.5 mmol) at the same temperature. The reaction mixture was stirred at -78°C for 1 hr and then quenched with water. After removal of the solvent, the residue was extracted with CH₂Cl₂. The organic layer was washed with water and brine, dried over MgSO₄ and concentrated in vacuo. The resulting residue was purified by chromatograph on silica gel to give 2-(5-methylthio-2-methoxyphenyl)-1,3-dioxolane (34.45 g, 77.2 %).
¹H NMR (CDCl₃) δ: 7.53 (d, J=2.6Hz, 1H), 7.30 (dd, J=8.4, 2.6Hz, 1H), 6.85 (d, J=8.4Hz, 1H), 6.12 (s, 1H), 4.17-4.00 (m, 4H), 3.85 (s, 3H), 2.45 (s, 3H).

The solution of 2-(5-methylthio-2-methoxyphenyl)-1,3-dioxolane (34.45 g, 152.24 mmol) in 1N-HCl (35 ml)/acetone (700 ml) was stirred at room temperature for 3 hr. After removal of the solvent, water was added to the residue and the mixture was extracted with CH₂Cl₂. The combined organic layer was washed with sat. NaHCO₃ and brine, dried over MgSO₄, concentrated in vacuo and distilled to give 5-methylthio-o-anisaldehyde (24.1 g, 86.7%) as a yellow oil.
¹H NMR (CDCl₃) δ: 10.4 (s, 1H), 7.72 (d, J=2.6Hz, 1H), 7.48 (dd, J=8.8, 2.6Hz, 1H), 6.94 (d, J=8.8Hz, 1H), 3.91 (s, 3H), 2.46 (s, 3H).

To the mixture of 5-methylthio-o-anisaldehyde (3.0 g, 16.5 mmol) in acetone (30 ml) and water (15 ml) was added 4-methylmorpholine-N-oxide (85.8 g, 49.5 mmol) and 0.18M-osmium tetraoxide aqueous solution (0.95 ml, 0.17 mmol) at room tempera-ture and was allowed to stir for 6 hr. The mixture was quenched by addition of aqueous sodium bisulfite (10 ml). After extraction of the quenched reaction mixture with CH₂Cl₂, the combined organic layer was washed with 1 N-sodium bisulfate and brine, dried over MgSO₄, filtered and concentrated in vacuo. Flash silica gel chromatography of the residue provide 5-methylsulfonyl-o-anisaldehyde (440 mg) as a colorless solid.
¹H NMR (CDCl₃) δ: 10.46 (s, 1H), 8.38 (d, J=2.6Hz, 1H), 8.13 (dd, J=8.8, 2.6Hz, 1H), 7.18 (d, J=8.8Hz, 1H), 4.06 (s, 3H), 3.07 (s, 3H).

### Synthesis of (3R,4S,5S,6S)-6-Diphenylmethyl-5-(-2-methoxy-5-methylsulfonylbenzyl-amino)-1-azabicyclo[2.2.2]octane-3-carboxamide dihydrochloride

A mixture of 5-methylsulfonyl-o-anisaldehyde (364 mg, 1.7 mmol), (3R,4S,5S,6S)-5-amino-6-diphenylmethyl-1-azabicyclo[2.2.2]octane-3-carboxamide (500 mg, 1.50 mmol) and NaBH(Oac)₃ (636 mg, 3.0 mmol) in CH₂Cl₂ (10 ml) was stirred at room temperature for 3 hr. The reaction mixture was extracted with CH₂Cl₂ (300 ml), washed with NaHCO₃ aqueous solution and brine, dried over MgSO₄ and concentrated in vacuo. The residue was purified by silica gel chromatography. The resulted product was diluted with HCl/methanol (15 ml), stirred for 30 min and the white precipitate (appeared during the reaction) was collected by filtration to afford a titled compound (589 mg, 58.2%) as a white crystalline.
m.p.: 205-208 °C (decomp.)
IR (KBr) : 3045, 3020, 1685, 1605, 1495, 1455, 1320, 1260, 1130, 1020, 980, 765, 715 cm⁻¹.
¹H NMR (CDCl₃, free amine) δ: 7.81 (dd, J = 2.6, 8.8 Hz, 1H), 7.54 (d, J = 2.6 Hz, 1H), 7.31 - 7.07 (m, 10H), 6.82 (d, J = 8.8 Hz, 1H), 5.73 (br. s, 1H), 5.40 (br. s, 1H), 4.46 (d, J = 12.1 Hz, 1H), 3.77 (d, J = 13.6 Hz, 1H), 3.65 - 3.61 (m, 1H), 3.57 (s, 3H), 3.27 (d, J = 13.6 Hz, 1H), 3.20 - 3.08 (m, 2H), 3.03 (s, 3H), 2.92 - 2.66 (m, 3H), 2.44 - 2.37 (m, 2H), 1.76 - 1.72 (m, 2H),

### Comparative Example 2

### (3R,4S,5S,6S)-6-Diphenylmethyl-5-(-2-methoxy-5-methylsulfonylbenzylamino)-1-azabicyclo[2.2.2]octane-3-carboxylic acid dihydrochloride

A solution of (3R,4S,5S,6S)-6-Diphenylmethyl-5-(-2- methoxy-5-methylsulfonylbenzylamino)-1-azabicyclo[2.2.2]octane-3-carboxamidedihydrochloride(thecompound of Example 1, 400 mg) in conc. HCI (10 ml) was warmed up to 90°C and stirred for 15 hr. After cooling down to room temperature, the mixture was evaporated in vacuo. The residue was recrystallized from EtOAc-MeOH to give a titled compound (268.2 mg, 55.6%) as a white crystalline.
m.p.: 208-211 °C (decomp.)
IR (Kbr): 3040, 1725, 1605, 1455, 1400, 1360, 1295, 1130, 1020, 965, 760, 710, 620 cm⁻¹.
¹H NMR (CDCl₃, free amine) δ: 7.77 (dd, J = 2.1, 8.6 Hz, 1H), 7.48 (d, J = 2.1 Hz, 1H), 7.41 - 7.07 (m, 10H), 6.80 (d, J = 8.6 Hz, 1H), 4.49 (d, J = 12.1 Hz, 1H), 4.04 (br.t, 1H), 3.60 (s, 3H), 3.01 (s, 3H), 2.56 (m, 2H), 1.87 (m, 2H), 1.60 (m, 2H).

### Comparative Example 3

### (3R,4S,5S,6S)-6-Diphenylmethyl-5-[2-methoxy-5-(N-methyl-N-methylsulfonylamino)benzylamino]-1-azabicyclo[2.2.2]octane-3-carboxamide dihydrochloride

### Synthesis of 5-(N-methyl-N-methylsulfonylamino)-o-anisaldehyde

To a solution of 5-nitrosalicylaldehyde (1.00 g, 6.0 mmol) in DMF (20 ml) was added NaH (246 mg) and methyl iodide (2.56 g, 18 mmol) at 0°C. The mixture was stirred for 8.5 hr at 60°C. After the reaction mixture was extracted with ether, the extract was washed with aqueous NaHCO₃, brine and 5%-HCl/aq.NaCl, dried over MgSO₄ and concentrated in vacuo to afford 2-methoxy-5-nitrobenzaldehyde (0.95 g, 87.8%) as a yellow needle.
¹H NMR (CDCl₃) δ: 10.45 (s, 1H), 8.70 (d, J=2.9Hz, 1H), 8.45 (dd, J=8.8, 2.9Hz, 1H), 7.14 (d, J=8.8Hz, 1H), 4.08 (s, 3H).

A solution of 2-methoxy-5-nitrobenzaldehyde (100 g, 550 mmol), ethylene glycol (36.9 ml, 660 mmol) and CSA (5.00 g) was heated at reflux under dehydrating condition for 5 hr. After removal of the solvent, triethylamine (6 ml) was added to the residue and the mixture was recrystallized from first ethyl acetate and from next isopropanol to give 2-(2-methoxy-5-nitrophenyl)-1,3-dioxolane (99.7 g, 80.5%) as a yellow solid.
¹H NMR (CDCl₃) δ: 8.44 (d, J=2.9Hz, 1H), 8.26 (dd, J=8.8, 2.9Hz, 1H), 6.98 (d, J=8.8Hz, 1H), 6.14 (s, 1H), 4.20-4.03 (m, 4H), 3.97 (s, 3H).

To a solution of 2-(2-methoxy-5-nitrophenyl)-1,3-dioxolane (1.00 g, 4.4 mmol) in methanol (45 ml) was added PtO₂ (20 mg) as a catalyst. The resulted mixture was reacted with Parr Shaker (2.2 kg/cm₂) for 1.5 hr. After removal of catalyst by filtration, the reaction mixture was evaporated in vacuo to give crude 2-(5-amino-2-methoxyphenyl)-1,3-dioxolane (894.6 mg, 100%) as a brown oil.
¹H NMR (CDCl₃) δ: 6.92 (d, J=2.9Hz, 1H), 6.75 (d, J=8.8Hz, 1H), 6.66 (dd, J=8.8, 2.9Hz, 1H), 6.09 (s, 1H), 4.16-3.99 (m, 4H), 3.79 (s, 3H), 3.45 (s, 2H).

To a solution of 2-(5-amino-2-methoxyphenyl)-1,3-dioxolane (867 mg, 4.4 mmol) in pyridine (2 ml) was added methanesulfonyl chloride (375 µl, 4.84 mmol) and stirred for 20 hr. To the resulted reaction mixture was added water and aqueous NaHCO₃, and then extracted with ether/CH₂Cl₂. The extract was evaporated in vacuo to give crude 2-(5-methylsulfonylamino-2-methoxyphenyl)-1,3-dioxolane (1.06 g, 88.1%) as a slight brown oil.
¹H NMR (CDCl₃) δ: 7.39 (d, J=2.9Hz, 1H), 7.32 (dd, J=8.8, 2.9Hz, 1H), 6.89 (d, J=8.8Hz, 1H), 6.62 (s, 1H), 6.11 (s, 1H), 4.18-4.00 (m, 4H), 3.86 (s, 3H), 2.94 (s, 3H).

To a solution of 2-(5-methylsulfonylamino-2-methoxyphenyl)-1,3-dioxolane (8.63 g, 31.6 mmol) in DMF (30 ml) was added NaH (1.51 g, 37.9 mmol) and methyl iodide (2.36 ml, 37.9 mmol) and then stirred at 60°C for 1 hr. After aqueous ammonium chloride was added to the reaction mixture, the mixture was extracted with ether/CH₂Cl₂. The extract was dried over MgSO₄ and evaporated in vacuo to give crude 2-[5-(N-methyl-N-methylsulfonylamino)-2-methoxyphenyl]-1,3-dioxolane (10.29 g) as a brown oil.
¹H NMR (CDCl₃) δ: 7.44 (d, J=2.9Hz, 1H), 7.30 (dd, J=8.8, 2.9Hz, 1H), 6.84 (d, J=8.8Hz, 1H), 6.02 (s, 1H), 4.07-3.95 (m, 4H), 3.81 (s, 3H), 3.22 (s, 3H), 2.76 (s, 3H).

A solution of 2-[5-(N-methyl-N-methylsulfonylamino)-2-methoxyphenyl]-1,3-dioxolane (4.0 g, 13.9 mmol) in 1N-HCl (4 ml)/ acetone (80 ml) was stirred at room temperature for 2 hr. After removal of the solvent, water was added to the residue and the mixture was extracted with CH₂Cl₂. The extract was washed with brine, dried over MgSO₄ and evaporated in vacuo to give crude 5-(N-5-methyl-N-methylsulfonylamino)-o-anisaldehyde (4.0 g, 118%). The resulted crude compound was recrystallized from ethyl acetate/hexane to give 5-(N-methyl-N-methylsulfonylamino)-o-anisaldehyde (2.54 g, 81%).
¹H NMR (CDCl₃) δ: 10.44 (s, 1H), 7.75 (d, J=2.6Hz, 1H), 7.67 (dd, J=8.8, 2.6Hz, 1H), 7.03 (d, J=8.8Hz, 1H), 3.96 (s, 3H), 3.31 (s, 3H), 2.84 (s, 3H).

### (3R,4S,5S,6S)-6-Diphenylmethyl-5-[2-methoxy-5-(N-methyl-N-methylsulfonylamino)benzylamino]-1-azabicyclo[2.2.2]octane-3-carboxamide dihydrochloride

This compound was prepared according to a similar manner with Comparative Example 1 from (3R,4S,5S,6S)-5-amino-6-diphenylmethyl-1-azabicyclo[2.2.2]octane-3-carboxamide and 5-(N-methyl-N-methylsulfonylamino)-o-anisaldehyde.
m.p.: 188.3 - 189.1 °C
IR (KBr) : 3435, 1685, 1678, 1502, 1458, 1331, 1246, 1142, 1022, 962 cm⁻¹.
¹H NMR (CDCl₃, free amine) δ: 7.35-7.05 (m, 12H), 6.64 (d, J = 8.8 Hz, 1H), 6.34 (br. s, 1H), 5.26 (br. s, 1H), 4.50 (d, J = 12.1 Hz, 1H), 3.76 ( d, J = 13.9 Hz, 1H), 3.7-3.55 (m, 1H), 3.40 (s, 3H), 3.35-3.0 (m, 3H), 3.26 (s, 3H), 2.95 - 2.8 (m, 2H), 2.89 (s, 3H), 2.8 - 2.6 (m, 1H), 2.5-2.2 (m, 2H), 1.8 - 1.5 (m, 2H).

### Comparative Example 4

### (3R,4S,5S,6S)-5-[5-(N-Acetyl-N-methylamino)-2-methoxybenzylamino]-6-diphenyl-methyl-1-azabicyclo[2.2.2]octane-3-carboxamide dihydrochloride

### Synthesis of 5-(N-acetyl-N-methylamino)-o-anisaldehyde

To a solution of 2-(5-amino-2-methoxyphenyl)-1,3-dioxolane (an intermediate for the compound of Comparative Example 3, 17.3 g, 88.8 mmol) and triethylamine (27 ml, 195.4 mmol) in CH₂Cl₂ (30 ml) was added dropwise acetic anhydride (9.3 ml, 97.7 mmol) and then stirred for 20 hr. To the mixture was added brine and then extracted with CH₂Cl₂, dried over MgSO₄ and concentrated in vacuo to give 2-(5-acetylamino-2-methoxyphenyl)-1,3-dioxolane (21.3 g, > 100%) as a brown solid.
¹H NMR (CDCl₃) δ: 7.71 (s, 1H), 7.64 (dd, J=8.8, 2.9Hz, 1H), 7.47 (d, J=2.9Hz, 1H), 6.84 (d, J=8.8Hz, 1H), 6.11 (s, 1H), 4.13-3.98 (m, 4H), 3.81 (s, 3H), 2.12 (s, 3H).

A solution of 2-(5-acetylamino-2-methoxyphenyl)-1,3-dioxolane (5.0 g, 21.1 mmol) in DMF (16 ml) was added to a suspension of NaH (1.0 g, 25.3 mmol) in DMF (5 ml) and then the mixture was stirred at room temperature for 10 min. To the mixture was added methyl iodide (3.6 g, 25.3 mmol) and then stirred at room tempera-ture for 1 hr. And to the resulted reaction mixture was added ammonium chloride and then extracted with CH₂Cl₂. The extract was washed with water and brine, dried over Na₂SO₄ and concentrated in vacuo to give crude 2-[5-(N-acetyl-N-methylamino)-2-methoxyphenyl]-1,3-dioxolane (5.0 g, 94%) as a orange oil.
¹H NMR (CDCl₃) δ: 7.38 (d, J=2.6Hz, 1H), 7.15 (dd, J=8.8, 2.6Hz, 1H), 6.93 (d, J=8.8Hz, 1H), 6.12 (s, 1H), 4.15-4.00 (m, 4H), 3.90 (s, 3H), 3.23 (s, 3H), 1.93 (s, 3H).

A solution of 2-[5-(N-acetyl-N-methylamino)-2-methoxyphenyl]-1,3-dioxolane (5.0 g) in 1N-HCl (4 ml)/acetone (80 ml) was stirred at room temperature for 1.5 hr. After removal of the solvent, water was added to the residue and the mixture was extracted with CHCl₃. The extract was washed with brine, dried over Na₂SO₄ and concentrated in vacuo to give a crude titled compound (3.1 g, 75%). The resulted crude product was recrystallized from ethyl acetate/hexane to afford the titled compound (2.18 g, 53%) as a brown solid.
¹H NMR (CDCl₃) δ: 10.46 (s, 1H), 7.66 (d, J=2.6Hz, 1H), 7.38 (dd, J=8.8, 2.6Hz, 1H), 7.05 (d, J=8.8Hz, 1H), 3.98 (s, 3H), 3.23 (s, 3H), 1.86 (s, 3H).

### Synthesis of (3R,4S,5S,6S)-5-[5-(N-acetyl-N-methylamino)-2-methoxybenzylamino] -6-diphenylmethyl-1-azabicyclo[2.2.2]octane-3-carboxamide dihydrochloride

This compound was prepared according to a similar manner with Comparative Example 1 from (3R,4S,5S,6S)-5-amino-6-diphenylmethyl-1-azabicyclo[2.2.2]octane-3-carboxamide and 5-(N-acetyl-N-methylamino)-o-anisaldehyde.
m.p. : 195.2-196.0 °C
IR (Kbr) : 3410, 1685, 1679, 1632, 1505, 1455, 1391, 1249, 1023 cm⁻¹.
¹H NMR (CDCl₃, free amine): 7.35-7.05 (m, 10H), 6.96 (dd, J = 2.6, 8.8 Hz, 1H), 6.73 (d, J = 8.8 Hz, 1H), 6.34 (d, J = 2.6 Hz, 1H), 5.54 (br. s, 1H), 5.47 (br. s, 1H), 4.46 (d, J = 11.7 Hz, 1H), 3.85-3.65 (m, 2H), 3.66 (s, 3H), 3.36 (d, J = 11.4 Hz, 1H), 3.30-3.00 (m, 4H), 3.20 (s, 3H), 2.80-2.65 (m, 1H), 2.60-2.40 (m, 2H), 1.90-1.60 (m, 2H), 1.81 (s, 3H).

### Example 1

### (3R,4S,5S,6S)-6-Diphenylmethyl-5-(5-Isopropenyl-2-methoxy)benzylamino-1-aza-bicyclo[2.2.2]octane-3-carboxamlde

### Synthesis of 5-lsopropenyl-o-anisaldehyde

5-Isopropenyl-o-anisaldehyde was prepared by J. K. Stille's method [Journal of Organic Chemistry, 52, 422 (1987)].

Tri-n-butyl-isopropenyltin (12.1 g, 36.6 mmol) dissolved in toluene (10 ml) was added to a mixture of 5-bromo-o-anisaldehyde (6.00 g, 27.9 mmol), tetrakis (triphenylphosphine)palladium (1.21 g, 1.05 mmol), and 2,6-t-butyl-4-methylphenol (10 mg) in toluene (50 ml) under nitrogen at room temperature. This mixture was heated at reflux for 7 hr. Ether and aq. KF solution (80 ml) were added to the reaction mixture, and the resulting solution was stirred for 3 hr. Insoluble materials were removed by filtration through Celite. The filtrate was extracted with ether, and the organic phase was washed with 1N-NaHSO₄, aq. NaHCO₃ and brine. The extracts were dried over Na₂SO₄, and concentrated by evaporation. The residual oil was purified by chromatography (SiO₂, 140 g, 5%-EtOAc/hexane) to afford 5-isopropenyl-o-anis-aldehyde, as a yellow oil (2.49 g, 51%).
¹H NMR (CDCl₃) δ: 10.47 (s, 1H), 7.93 (d, J = 2.6 Hz, 1H), 7.69 (dd, J = 8.8, 2.6 Hz, 1H), 6.96 (d, J = 8.8 Hz, 1H), 5.35 (br. s, 1H), 5.07 (m, 1H), 3.94 (s, 3H), 2.15 (m, 3H).

The said tri-n-butyl-isopropenyltin was prepared according to D. Seyferth's method [Journal of American Chemical Society, 79, 515 (1957)].

### Synthesis of (3R,4S,5S,6S)-6-Diphenylmethyl-5-(5-isopropenyl-2-methoxy)benzyl-amino-1-azabicyclo[2.2.2]octane-3-carboxamide

This compound was prepared according to a similar manner with Comparative Example 1 from (3R,4S,5S,6S)-5-amino-6-diphenylmethyl-1-azabicyclo[2.2.2]octane-3-carboxamide and 5-isopropenyl-o-anisaldehyde.
m.p.: 223 - 237 °C (decomp.)
IR (nujol): 3300, 3200, 3150, 1685, 1655 cm⁻¹.
¹H NMR (CDCl₃, free amine) δ: 7.35 - 7.10 (m, 11H), 6.94 (d, J = 2.2 Hz, 1H), 6.68 (d, J = 8.6 Hz, 1H), 5.37 (br. s, 2H), 5.22 (br. s, 1H), 5.00 (m, 1H), 4.47 (d, J = 12.2 Hz, 1H), 3.70 - 3.53 (m, 2H), 3.56 (s, 3H), 3.55 (d, J = 12.0 Hz, 1H), 3.35 - 2.95 (m, 3H), 3.23 (d, J = 12.0 Hz, 1H), 2.66 (br.t, J = 12.0 Hz, 1H), 2.52 - 2.40 (m, 2H), 2.10 (d, J = 0.50 Hz, 3H), 1.90 - 1.80 (m, 1H), 1.60 (m, 1H).

## Claims

1. A compound of the following chemical formula and its pharmaceutically acceptable salt: 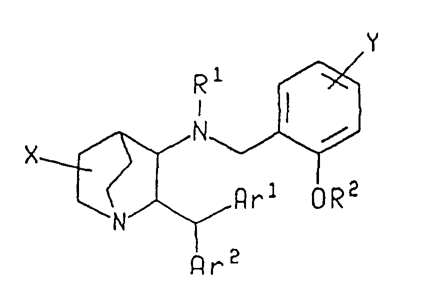 wherein Ar¹ and Ar² are each, independently, thienyl, phenyl, fluorophenyl, chlorophenyl or bromophenyl;
X is -CONR³R⁴, -CO₂R³, -CH₂OR³, -CH₂NR³R⁴ or -CONR³OR⁴;
R¹, R³ and R⁴ are each, independently, hydrogen or alkyl having 1 to 4 carbon atoms;
R² is alkyl having 1 to 4 carbon atoms;
Y is alkenyl having 2 to 4 carbon atoms;
with the proviso that when R¹ is hydrogen, and Ar¹ and Ar² are both phenyl, R² is not an alkyl group having 1 carbon atom.

2. A compound according to claim 1, wherein Ar¹ and Ar² are each phenyl.

3. A compound according to claim 2, wherein R² is methyl and R¹ is hydrogen.

4. A compound according to claim 3, wherein X is at the 3-position of the quinuclidine ring and X is carboxy or aminocarbonyl.

5. A compound according to claim 1 wherein Y is isopropenyl.

6. A compound according to claim 1, wherein the compound is (3R,4S,5S,6S)-5-(5-Isopropenyl-2-methoxybenzylamino)-6-diphenylmethyl-1-azabicyclo[2.2.2]octane-3-carboxamide;

7. A pharmaceutical composition for antagonizing substance P in a mammalian subject which comprises a therapeutically effective amount of a compound of claim 1 or its pharmaceutically acceptable salt together with a pharmaceutically acceptable carrier.

8. A pharmaceutical composition for the treatment of gastrointestinal or central nervous system disorders and the alleviation of inflammatory disease, asthma, pain or migraine in a mammalian subject which comprises a therapeuticalty effective amount of a compound of claim 1 or its pharmaceutically acceptable salt together with a pharmaceutically acceptable carrier.

9. Use of a compound of claim 1 in the preparation of a medicament for antagonizing substance P in a mammalian subject.

10. Use of a compound of claim 1 in the preparation of a medicament for treating a gastrointestinal or central nervous system disorders, or alleviating an inflammatory disease, asthma, pain or migraine, in a mammalian subject.

## Patentansprüche

1. Verbindung der nachstehenden chemischen Formel und ihr pharmazeutisch verträgliches Salz: 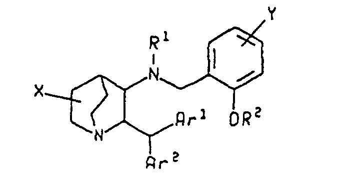 worin Ar¹ und Ar² jeweils unabhängig, Thienyl, Phenyl, Fluorphenyl, Chlorphenyl oder Bromphenyl darstellen;
X -CONR³R⁴, -CO₂R³, -CH₂OR³, -CH₂NR³R⁴ oder -CONR³OR⁴ darstellt;
R¹, R³ und R⁴ jeweils unabhängig Wasserstoff oder Alkyl mit 1 bis 4 Kohlenstoffatomen darstellen;
R² Alkyl mit 1 bis 4 Kohlenstoffatomen darstellt;
Y Alkenyl mit 2 bis 4 Kohlenstoffatomen darstellt;
mit der Maßgabe, dass wenn R¹ Wasserstoff darstellt und Ar¹ und Ar² beide Phenyl darstellen, R² keine Alkylgruppe mit 1 Kohlenstoffatom darstellt.

2. Verbindung nach Anspruch 1, worin Ar¹ und Ar² jeweils Phenyl darstellen.

3. Verbindung nach Anspruch 2, worin R² Methyl darstellt und R¹ Wasserstoff darstellt.

4. Verbindung nach Anspruch 3, worin X an der 3-Position des Chinuclidinrings vorliegt und X Carboxy oder Aminocarbonyl darstellt.

5. Verbindung nach Anspruch 1, worin Y Isopropenyl darstellt.

6. Verbindung nach Anspruch 1, wobei die Verbindung (3R,4S,5S,6S)-5-(5-Isopropenyl-2-methoxybenzylamino)-6-diphenylmethyl-1-azabicyclo[2.2.2]octan-3-carboxamid ist.

7. Pharmazeutische Zusammensetzung zum Entgegenwirken von Substanz P bei einem Säuger, die eine therapeutisch wirksame Menge einer Verbindung nach Anspruch 1 oder ihres pharmazeutisch verträglichen Salzes zusammen mit einem pharmazeutisch verträglichen Träger umfasst.

8. Pharmazeutische Zusammensetzung zur Behandlung von gastrointestinalen Störungen oder von Störungen des zentralen Nervensystems und zur Linderung von entzündlicher Erkrankung, Asthma, Schmerz oder Migräne bei einem Säuger, die eine therapeutisch wirksame Menge einer Verbindung nach Anspruch 1 oder ihres pharmazeutisch verträglichen Salzes zusammen mit einem pharmazeutisch verträglichen Träger umfasst.

9. Verwendung einer Verbindung nach Anspruch 1 bei der Herstellung eines Arzneimittels zum Entgegenwirken von Substanz P bei einem Säuger.

10. Verwendung einer Verbindung nach Anspruch 1 bei der Herstellung eines Arzneimittels zur Behandlung von gastrointestinalen Störungen oder von Störungen des zentralen Nervensystems oder zur Linderung einer entzündlichen Erkrankung, Asthma, Schmerz oder Migräne bei einem Säuger.

## Revendications

1. Composé répondant à la formule chimique suivante, et son sel pharmaceutiquement acceptable : 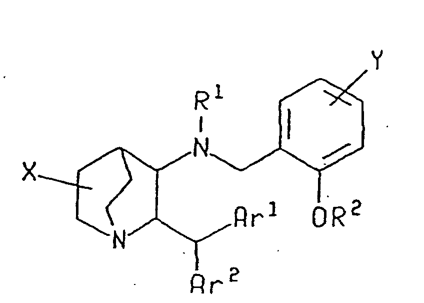 formule dans laquelle Ar¹ et Ar² représentent chacun, indépendamment, un groupe thiényle, phényle, fluorophényle, chlorophényle ou bromophényle ;
X représente un groupe -CONR³R⁴, -CO₂R³, -CH₂OR³, -CH₂NR³R⁴ ou -CONR³OR⁴ ;
R¹, R³ et R⁴ représentent chacun, indépendamment, un atome d'hydrogène ou un groupe alkyle ayant 1 à 4 atomes de carbone ;
R² représente un groupe alkyle ayant 1 à 4 atomes de carbone ;
Y représente un groupe alcényle ayant 2 à 4 atomes de carbone
sous réserve que, lorsque R¹ représente un atome d'hydrogène et Ar¹ et Ar² représentent l'un et l'autre un groupe phényle, R² ne représente pas un groupe alkyle ayant 1 atome de carbone.

2. Composé suivant la revendication 1, dans lequel Ar¹ et Ar² représentent chacun un groupe phényle.

3. Composé suivant la revendication 2, dans lequel R² représente un groupe méthyle et R¹ représente un atome d'hydrogène.

4. Composé suivant la revendication 3, dans lequel X est en position 3 du noyau quinuclidine et X représente un groupe carboxy ou aminocarbonyle.

5. Composé suivant la revendication 1, dans lequel Y représente un groupe isopropényle.

6. Composé suivant la revendication 1, qui est le (3R,4S,5S,6S)-5-(5-isopropényl-2-méthoxybenzylamino)-6-diphénylméthyl-1-azabicyclo[2.2.2]octane-3-carboxamide.

7. Composition pharmaceutique pour antagoniser la substance P chez un sujet consistant en un mammifère, qui comprend une quantité thérapeutiquement efficace d'un composé suivant la revendication 1 ou de son sel pharmaceutiquement acceptable conjointement avec un support pharmaceutiquement acceptable.

8. Composition pharmaceutique pour le traitement de troubles gastro-intestinaux ou de troubles du système nerveux central et pour le soulagement d'une maladie inflammatoire, de l'asthme, de la douleur ou de la migraine chez un sujet consistant en un mammifère, qui comprend une quantité thérapeutiquement efficace d'un composé suivant la revendication 1 ou de son sel pharmaceutiquement acceptable conjointement avec un support pharmaceutiquement acceptable.

9. Utilisation d'un composé suivant la revendication 1 dans la préparation d'un médicament destiné à antagoniser la substance P chez un sujet consistant en un mammifère.

10. Utilisation d'un composé suivant la revendication 1 dans la préparation d'un médicament destiné au traitement de troubles gastro-intestinaux ou de troubles du système nerveux central, ou au soulagement d'une maladie inflammatoire, de l'asthme, de la douleur ou de la migraine chez un sujet consistant en un mammifère.
